# EUROPEAN PATENT SPECIFICATION

(11) **EP 3 024 440 B1**
(45) Date of publication and mention of the grant of the patent: **28.11.2018**
(21) Application number: 14830224.3
(22) Date of filing: 22.07.2014
(51) Int. Cl.: A61K 9/14, A61K 9/20, A61K 31/421

(54) **FORMULATION OF METAXALONE**
METAXALONFORMULIERUNG
FORMULATION DE MÉTAXALONE

(30) Priority: 22.07.2013 US 201361857199 P
(43) Date of publication of application: 01.06.2016
(73) Proprietor: Iceutica Inc., Philidelphia, Pennsylvania 19112 (US)
(72) Inventor: BOSCH, H. William, Bryn Mawr, Pennsylvania 19010 (US)
(74) Representative: Kunz, Herbert
(86) International application number: PCT/US2014/047701
(87) International publication number: WO 2015/013336

(56) References cited:
- WO-A1-2005/016310
- WO-A1-2010/121320
- WO-A2-2009/019662
- US-A1- 2012 263 760
- US-B1- 7 714 006

## Description

### Field of the Disclosure

The present disclosure relates to methods for producing particles (e.g., nanoparticles) of metaxalone using dry milling processes as well as compositions comprising metaxalone, medicaments, including unit dosage forms, produced using metaxalone that is in nanoparticulate form and/or compositions.

### Background

Poor bioavailability is a significant problem encountered in the development of therapeutic compositions. Many factors affect bioavailability, including the form of dosage and the solubility and dissolution rate of the active material (drug substance). However, due to the complex interactions in the human body, the pharmacokinetic properties of a particular drug product (e.g., a particular dosage form) cannot be predicted based on the solubility of the drug substance.
Metaxalone is commercially marketed under the name Skelaxin® (King Pharmaceitucals, Inc.), which is indicated as an adjunct to rest, physical therapy, and other measures for the relief of discomfort associated with acute, painful musculoskeletal conditions. Skelaxin® is taken as an 800 mg tablet three to four times a day. Previous animal studies have shown that by reducing the size of metaxalone much higher rates of absorption and overall bioavaiability (as measured by AUC) can be achieved. However, such animal studies are not necessarily predictive of the pharmacokinetic properties on the drug product in humans.
US 2012/0263760 A1 describes methods for producing particles of metaxalone using dry milling processes as well as compositions comprising metaxalone, medicaments produced using metaxalone in particulate form and/or compositions, and to methods of treatment of an animal, including man, using a therapeutically effective amount of metaxalone administered by way of said medicaments.

### Summary

The present invention is defined by independent claims 1. The dependent claims depict other embodiments of the invention. Described herein are unit dosage forms of metaxalone (5-[(3,5-dimethylphenoxy) methyl]-2-oxazolidinone) containing 300 mg of metaxalone, wherein the dissolution rate of the metaxalone, when tested in a Sotax Dissolution Apparatus using 1000 ml of 0.01 N HCl (pH=2) at 37°C and Type 2 Apparatus (paddle) set to a rotational speed of 100 rpm, is such that at least 80% dissolves in 60 min.
In various embodiments: the unit dosage form (referred to as a submicron dosage form) comprises metaxalone having a median particle size, on a volume average basis, between 50 nm and 900 nm (e.g., less than 800 nm, 700 nm, 600 nm, 500 nm, 400 nm, or 300 nm, but greater than 50 nm or greater than 100 nm). In various embodiments, when the unit dosage form is tested in a Sotax Dissolution Apparatus using 1000 ml of 0.01 N HCl (pH=2) at 37°C and Type 2 Apparatus (paddle) set to a rotational speed of 100 rpm, the dissolution rate of the metaxaloen is such that: at least 90% of the metaxalone dissolves in 60 min; at least 99% of the metaxalone dissolves in 60 min; at least 50% of the metaxalone dissolves in 30 min; at least 50% of the metaxalone dissolves in 20 min; at least 50% of the metaxalone dissolves in 15 min; at least 25% of the metaxalone dissolves in 20 min; at least 25% of the metaxalone dissolves in 15 min; at least 25% of the metaxalone dissolves in 10 min; the unit dosage form is a tablet (e.g., a compressed tablet); the unit dosage form contains 300 mg of metaxalone. In some cases two unit dosage forms are administered for a total dose of 600 mg of metaxalone and this total dose is administered 2, 3 or 4 times daily. In various embodiments of the unit dosage form: the mean Cmax when administered to female subjects is no greater than 140%, 130%, 120%, or 110% of the mean Cmax when administered to male subjects, when the unit dosage form is administered in the fasted state; the mean Cmax when administered to female subjects is no greater than 120% of the mean Cmax when administered to male subjects, when the unit dosage form is administered in the fasted state; the mean AUC∞ when administered to female subjects is no greater than 140%, 130%, 120%, or 110% of the mean AUC∞ when administered to male subjects, when the unit dosage form is administered in the fasted state; the mean AUC∞ when administered to female subjects is no greater than 120% of the mean AUC∞ when administered to male subjects, when the unit dosage form is administered in the fasted state; the mean AUC₁₋ₜ when administered to female subjects is no greater than 140%, 130%, 120%, or 110% of the mean AUC₁₋ₜ when administered to male subjects, when the unit dosage form is administered in the fasted state; the mean AUC₁₋ₜ when administered to female subjects is no greater than 120% of the mean AUC₁₋ₜ when administered to male subjects, when the unit dosage form is administered in the fasted state; the mean Tmax when administered to female subjects is no greater than 140%, 130%, 120% or 110% of the mean Tmax when administered to male subjects, when the unit dosage form is administered in the fasted state: the mean Tmax when administered to female subjects is no greater than 120% of the mean Tmax when administered to male subjects, when the unit dosage form is administered in the fasted state; the mean T_{1/2} when administered to female subjects is no greater than 140%, 130%, 120% or 110% of the mean T_{1/2} when administered to male subjects, when the unit dosage form is administered in the fasted state; the mean T_{1/2} when administered to female subjects is no greater than 120% of the mean T_{1/2} when administered to male subjects, when the unit dosage form is administered in the fasted state. In some cases there is no clicinally significant difference in the Cmax or AUC_{1-∞} between female and male subjects with the unit dose is administered in the fasted state.

Iin some embodiments: the ratio of the geometric mean Cmax in the fed state versus the fasted state is between 0.8 and 1.2; the ratio of the geometric mean Cmax in the fed state versus the fasted state is between 0.8 and 1.0; the ratio of the geometric mean Cmax in the fed state versus the fasted state is between 0.8 and 0.9; the ratio of the geometric mean AUC1-∞ in the fed state versus the fasted state is between 0.8 and 1.2; the ratio of the geometric mean AUC1-∞ in the fed state versus the fasted state is between 0.8 and 1.0; the ratio of the geometric mean AUC1-∞ in the fed state versus the fasted state is between 0.8 and 0.9; the ratio of the geometric mean AUC1-t in the fed state versus the fasted state is between 0.8 and 1.2; the ratio of the geometric mean AUC1-t in the fed state versus the fasted state is between 0.8 and 1.0; the ratio of the geometric mean AUC1-t in the fed state versus the fasted state is between 0.8 and 0.9; the ratio of the geometric mean T_{1/2} in the fed state versus the fasted state is between 0.8 and 1.2; the ratio of the geometric mean T_{1/2} in the fed state versus the fasted state is between 0.8 and 1.0; and the ratio of the geometric mean T_{1/2} in the fed state versus the fasted state is between 0.8 and 0.9.

In some embodiments of the unit dosage form: the geometric mean coefficient of variation in Cmax in the fasted state is less than 40%, 35%, 30%, 25%, or 20%; the geometric mean coefficient of variation in AUC∞ in the fasted state is less than 40%, 35%, 30%, 25%, or 20%; the geometric mean coefficient of variation in T_{1/2} in the fasted state is less than 40%, 35%, 30%, 25%, or 20%; the geometric mean coefficient of variation in Cmax in the fed state is less than 40%, 35%, 30%, 25%, or 20%; the geometric mean coefficient of variation in AUC1-∞ in the fed state is less than 40%, 35%, 30%, 25%, or 20%; the geometric mean coefficient of variation in T_{1/2} in the fed state is less than 40%, 35%, 30%, 25%, or 20%; the mean AUC1-∞ per mg of metaxalone in the fasted state is 80% to 125% of 18.7 ng·h/mL; the mean AUC1-∞ per mg of metaxalone in the fasted state is 80% to 125% of 18.8 ng·h/mL; the mean AUC∞ in the fasted stated is 80%-125% of 7479 ng·h/mL when a total dose selected from 200, 225, 250, 275, 300, 325, 350, 375, 400, 425, 450, 475, 500, 525, 550, 575, 600 or 625 mg is administered; the mean AUC1-∞ in the fasted stated is 80%-125% of 15044 ng·h/mL when a total dose selected from 200, 225, 250, 275, 300, 325, 350, 375, 400, 425, 450, 475, 500, 525, 550, 575, 600 or 625 mg is administered; the mean Cmax in the fasted state is greater (e.g., at least 10%, 20%, 30%, 40%, 50%, 60%, 70% or 80% greater) than 983 ng/mL at a total dose that provides a mean AUC∞ in the fasted stated is 80%-125% of 7479 ng·h/mL; the mean Cmax in the fasted state is greater (e.g., at least 10%, 20%, 30%, 40%, 50%, 60%, 70% or 80% greater) than 1816 ng/mL at a total dose that provides a mean AUC1-∞ in the fasted stated is 80%-125% of 15044 ng·h/mL; the Tmax in the fasted state is less than 2.7 hrs, 2.5 hrs, 2.3 hrs, 2.1 hrs, 1.9 hrs or 1.7 hrs; and the Tmax in the fed state is less than 2.7 hrs, 2.5 hrs, 2.3 hrs, 2.1 hrs, 1.9 hrs or 1.7 hrs.

Unless specified, the term "mean" in the context of Cmax, AUC, Tmax and other pharmacokinetic parameters refers to the geometric mean unless specified otherwise. Unless otherwise specified mean pharmacokinetic parameters are recited at the 90% confidence interval. Cmax is recited in ng/ml; AUC is in ng·hr/mL; and Tmax and T1/2 are in hrs. The fed state refers to administration after a standard high fat meal.

In one preferred embodiment, the median particle size, determined on a particle volume basis, is equal to or less than a size selected from the group 900 nm, 800 nm, 700 nm, 600nm, 500nm, 400 nm, 300nm, 200nm and 100 nm. In some cases, median particle size, determined on a particle volume basis, is greater than 25nm, 50 nm, or 100 nm. In some cases, the median particle size is between 900 and 100, 800 and 100, 700 and 100, 600 and 100, 500 and 100, or 400 and 100 nm.

Throughout this specification, unless the context requires otherwise, the word "comprise" or variations, such as "comprises" or "comprising" will be understood to imply the inclusion of a stated integer, or group of integers, but not the exclusion of any other integers or group of integers. It is also noted that in this disclosure, and particularly in the claims and/or paragraphs, terms such as "comprises", "comprised", "comprising" and the like can have the meaning attributed to it in US Patent law; e.g., they can mean "includes", "included", "including", and the like.

"Therapeutically effective amount" as used herein with respect to methods of treatment and in particular drug dosage, shall mean that dosage that provides the specific pharmacological response for which the drug is administered in a significant number of subjects in need of such treatment. It is emphasized that "therapeutically effective amount," administered to a particular subject in a particular instance will not always be effective in treating the diseases described herein, even though such dosage is deemed a "therapeutically effective amount" by those skilled in the art. It is to be further understood that drug dosages are, in particular instances, measured as oral dosages.

There are a wide range of techniques that can be utilized to characterize the particle size of a material. Those skilled in the art also understand that almost all these techniques do not physically measure the actually particle size, as one might measure something with a ruler, but measure a physical phenomena which is interpreted to indicate a particle size. As part of the interpretation process some assumptions need to be made to enable mathematical calculations to be made. These assumptions deliver results such as an equivalent spherical particle size, or a hydrodynamic radius.

Amongst these various methods, two types of measurements are most commonly used. Photon correlation spectroscopy (PCS), also known as 'dynamic light scattering' (DLS), is commonly used to measure particles with a size less than 10 micron. Typically this measurement yields an equivalent hydrodynamic radius often expressed as the average size of a number distribution. The other common particle size measurement is laser diffraction which is commonly used to measure particle size from 100 nm to 2000 micron. This technique calculates a volume distribution of equivalent spherical particles that can be expressed using descriptors such as the median particle size or the % of particles under a given size.

Those skilled in the art recognize that different characterization techniques such as photon correlation spectroscopy and laser diffraction measure different properties of a particle ensemble. As a result multiple techniques will give multiple answers to the question, "what is the particle size." In theory one could convert and compare the various parameters each technique measures, however, for real world particle systems this is not practical. As a result the particle size used to describe this invention will be given as two different sets of values that each relate to these two common measurement techniques, such that measurements could be made with either technique and then evaluated against the description of this invention. For measurements made using a photo correlation spectroscopy instrument, or an equivalent method known in the art, the term "number average particle size" is defined as the average particle diameter as determined on a number basis.

For measurements made using a laser diffraction instrument, or an equivalent method known in the art, the term "median particle size" is defined as the median particle diameter as determined on an equivalent spherical particle volume basis. Where the term median is used, it is understood to describe the particle size that divides the population in half such that 50 % of the population is greater than or less than this size. The median particle size is often written as D50, D(0.50) or D[0.5] or similar. As used herein D50, D(0.50) or D[0.5] or similar shall be taken to mean "median particle size".

The term "Dx of the particle size distribution" refers to the xth percentile of the distribution; thus, D90 refers to the 90^{th} percentile, D95 refers to the 95^{th} percentile, and so forth. Taking D90 as an example this can often be written as, D(0.90) or D[0.9] or simialr. With respect to the median particle size and Dx an upper case D or lowercase d are interchangeable and have the same meaning.

Another commonly used way of describing a particle size distribution measured by laser diffraction, or an equivalent method known in the art, is to describe what % of a distribution is under or over a nominated size. The term "percentage less than" also written as "%<" is defined as the percentage, by volume, of a particle size distribution under a nominated size -for example the % < 1000 nm. The term "percentage greater than" also written as "%>" is defined as the percentage, by volume, of a particle size distribution over a nominated size, for example the % > 1000 nm.

Suitable methods to measure an accurate particle size where the active material has substantive aqueous solubility or the matrix has low solubility in a water-based dispersant are outlined below.
1. In the circumstance where insoluble matrix such as microcrystalline cellulose prevents the measurement of the active material separation techniques such as filtration or centrifugation could be used to separate the insoluble matrix from the active material particles. Other ancillary techniques would also be required to determine if any active material was removed by the separation technique so that this could be taken into account.
2. In the case where the active material is too soluble in water other solvents could be evaluated for the measurement of particle size. Where a solvent could be found that active material is poorly soluble in but is a good solvent for the matrix a measurement would be relatively straight forward. If such a solvent is difficult to find another approach would be to measure the ensemble of matrix and active material in a solvent (such as iso-octane) which both are insoluble in. Then the powder would be measured in another solvent where the active material is soluble but the matrix is not. Thus with a measurement of the matrix particle size and a measurement of the size of the matrix and active material together an understanding of the active material particle size can be obtained.
3. In some circumstances image analysis could be used to obtain information about the particle size distribution of the active material. Suitable image measurement techniques might include transmission electron microscopy (TEM), scanning electron microscopy (SEM), optical microscopy and confocal microscopy. In addition to these standard techniques some additional technique would be required to be used in parallel to differentiate the active material and matrix particles. Depending on the chemical makeup of the materials involved possible techniques could be elemental analysis, raman spectroscopy, FTIR spectroscopy or fluorescence spectroscopy.

Where the particles of the active ingredient are relatively insoluble in water and are dispersed in material that is realtively soluble in water, the more soluble materials can be dissolved in water permitting recovery and size measurement of the relatively insoluble active ingredient.

Throughout this specification, unless the context requires otherwise, the phrase "dry mill" or variations, such as "dry milling", should be understood to refer to milling in at least the substantial absence of liquids. If liquids are present, they are present in such amounts that the contents of the mill retain the characteristics of a dry powder.

"Flowable" means a powder having physical characteristics rendering it suitable for further processing using typical equipment used for the manufacture of pharmaceutical compositions and formulations.

The invention described herein may include one or more ranges of values (e.g. size, concentration etc). A range of values will be understood to include all values within the range, including the values defining the range.

Other definitions for selected terms used herein may be found within the detailed description of the invention and apply throughout. Unless otherwise defined, all other scientific and technical terms used herein have the same meaning as commonly understood to one of ordinary skill in the art to which the invention belongs.

Inclusion of a reference does not constitute an admission is made that any of the references constitute prior art or are part of the common general knowledge of those working in the field to which this invention relates.

### Figures

Figure 1 depicts the size distribution of milled and unmilled metaxalone particles.
Figure 2 depicts the results of an analysis of dissolution of metaxalone in submicron tablets and Skelaxin®.
Figure 3 depict the results of dynamic vapor sorption analysis of submicron tablets

### Detailed Description

The metaxalone particles incorporated into the submicron unit dosage forms described herein can be produced usung a variety of methods. In some case there are prepared by dry milling metaxalone in a mill with milling bodies and a grinding matrix. The grinding matrix includes one or more millable grinding compound such a lactose or mannitol and a surfactant (e.g. sodium lauryl sulfate).

### Dry Milling

In some embodiments of the dry milling process, metaxalone, grinding matrix, in the form of crystals, powders, or the like, are combined in suitable proportions with the plurality of milling bodies in a milling chamber that is mechanically agitated (i.e. with or without stirring) for a predetermined period of time at a predetermined intensity of agitation. Typically, a milling apparatus is used to impart motion to the milling bodies by the external application of agitation, whereby various translational, rotational or inversion motions or combinations thereof are applied to the milling chamber and its contents, or by the internal application of agitation through a rotating shaft terminating in a blade, propeller, impeller or paddle or by a combination of both actions.

During milling, motion imparted to the milling bodies can result in application of shearing forces as well as multiple impacts or collisions having significant intensity between milling bodies and particles of the biologically active material and grinding matrix. The nature and intensity of the forces applied by the milling bodies to the metaxalone and the grinding matrix is influenced by a wide variety of processing parameters including: the type of milling apparatus; the intensity of the forces generated, the kinematic aspects of the process; the size, density, shape, and composition of the milling bodies; the weight ratio of the metaxalone and grinding matrix mixture to the milling bodies; the duration of milling; the physical properties of both the metaxalone and the grinding matrix; the atmosphere present during activation; and others.

Advantageously, the media mill is capable of repeatedly or continuously applying mechanical compressive forces and shear stress to the metaxalone and the grinding matrix. Suitable media mills include but are not limited to the following: high-energy ball, sand, bead or pearl mills, basket mill, planetary mill, vibratory action ball mill, multi-axial shaker/mixer, stirred ball mill, horizontal small media mill, multi-ring pulverizing mill, and the like, including small milling media. The milling apparatus also can contain one or more rotating shafts.

In a preferred form of the invention, the dry milling is performed in a ball mill. Throughout the remainder of the specification reference will be made to dry milling being carried out by way of a ball mill. Examples of this type of mill are attritor mills, nutating mills, tower mills, planetary mills, vibratory mills and gravity-dependent-type ball mills. It will be appreciated that dry milling in accordance with the method of the invention may also be achieved by any suitable means other than ball milling. For example, dry milling may also be achieved using jet mills, rod mills, roller mills or crusher mills.

In some embodiments, the milling time period is a range selected from the group consisting of: between 10 minutes and 2 hours, between 10 minutes and 90 minutes, between 10 minutes and 1 hour, between 10 minutes and 45 minutes, between 10 minutes and 30 minutes, between 5 minutes and 30 minutes, between 5 minutes and 20 minutes, between 2 minutes and 10 minutes, between 2 minutes and 5 minutes, between 1 minutes and 20 minutes, between 1 minute and 10 minutes, and between 1 minute and 5 minutes.

In some embodiments, the milling bodies comprise materials selected from the group consisting of: ceramics, glasses, polymers, ferromagnetics and metals. Preferably, the milling bodies are steel balls having a diameter selected from the group consisting of: between 1 and 20 mm, between 2 and 15 mm and between 3 and 10 mm. In another preferred embodiment, the milling bodies ares zirconium oxide balls having a diameter selected from the group consisting of: between 1 and 20 mm, between 2 and 15 mm and between 3 and 10 mm. Preferably, the dry milling apparatus is a mill selected from the group consisting of: attritor mills (horizontal or vertical), nutating mills, tower mills, pearl mills, planetary mills, vibratory mills, eccentric vibratory mills, gravity-dependent-type ball mills, rod mills, roller mills and crusher mills. Preferably, the milling medium within the milling apparatus is mechanically agitated by 1, 2 or 3 rotating shafts. Preferably, the method is configured to produce the biologically active material in a continuous fashion.

Preferably, the total combined amount of metaxalone and grinding matrix in the mill at any given time is equal to or greater than a mass selected from the group consisting of: 200 grams, 500 grams, 1 kg, 2kg, 5kg, 10kg, 20kg, 30kg, 50kg, 75kg, 100kg, 150kg, and 200kg. Preferably, the total combined amount of metaxalone and grinding matrix is less than 2000kg.

In some embodiments, the millable grinding compound is a single material or is a mixture of two or more materials in any proportion. Preferably, the single material or a mixture of two or more materials is selected from the group consisting of: mannitol, sorbitol, Isomalt, xylitol, maltitol, lactitol, erythritol, arabitol, ribitol, glucose, fructose, mannose, galactose, anhydrous lactose, lactose monohydrate, sucrose, maltose, trehalose, maltodextrins, dextrin, and inulin.

The milling matrix can also include a surfactant such as sodium lauryl sulphate.

During milling one or more of the following can be present: TAB, CTAC, Cetrimide, cetylpyridinium chloride, cetylpyridinium bromide, benzethonium chloride, PEG 40 stearate, PEG 100 stearate, poloxamer 188, poloxamer 338, poloxamer 407, polyoxyl 2 stearyl ether, polyoxyl 100 stearyl ether, polyoxyl 20 stearyl ether, polyoxyl 10 stearyl ether, polyoxyl 20 cetyl ether, polysorbate 20, polysorbate 40, polysorbate 60, polysorbate 61, polysorbate 65, polysorbate 80, polyoxyl 35 castor oil, polyoxyl 40 castor oil, polyoxyl 60 castor oil, polyoxyl 100 castor oil, polyoxyl 200 castor oil, polyoxyl 40 hydrogenated castor oil, polyoxyl 60 hydrogenated castor oil, polyoxyl 100 hydrogenated castor oil, polyoxyl 200 hydrogenated castor oil, cetostearyl alcohol, macrogel 15 hydroxystearate, sorbitan monopalmitate, sorbitan monostearate, sorbitan trioleate, Sucrose Palmitate, Sucrose Stearate, Sucrose Distearate, Sucrose laurate, Glycocholic acid, sodium Glycholate, Cholic Acid, Soidum Cholate, Sodium Deoxycholate, Deoxycholic acid, Sodium taurocholate, taurocholic acid, Sodium taurodeoxycholate, taurodeoxycholic acid, soy lecithin, phosphatidylcholine, phosphatidylethanolamine, phosphatidylserine, phosphatidylinositol, PEG4000, PEG6000, PEG8000, PEG10000, PEG20000, alkyl naphthalene sulfonate condensate/Lignosulfonate blend,Calcium Dodecylbenzene Sulfonate, Sodium Dodecylbenzene Sulfonate,Diisopropyl naphthaenesulphonate, erythritol distearate, Naphthalene Sulfonate Formaldehyde Condensate, nonylphenol ethoxylate (poe-30), Tristyrylphenol Ethoxylate, Polyoxyethylene (15) tallowalkylamines, sodium alkyl naphthalene sulfonate, sodium alkyl naphthalene sulfonate condensate, sodium alkylbenzene sulfonate, sodium isopropyl naphthalene sulfonate, Sodium Methyl Naphthalene Formaldehyde Sulfonate, sodium n-butyl naphthalene sulfonate, tridecyl alcohol ethoxylate (poe-18), Triethanolamine isodecanol phosphate ester, Triethanolamine tristyrylphosphate ester, Tristyrylphenol Ethoxylate Sulfate, Bis(2-hydroxyethyl)tallowalkylamines.

Preferably, the millable grinding and the surfactant are selected from materials considered to be Generally Regarded as Safe (GRAS) for pharmaceutical products.

In some cases, the millable grinding compound is capable of being physically degraded under the dry milling conditions used to produce metaxalone particles. In one embodiment, after milling, the millable grinding compound is of a comparable particle size to the milled metaxalone. In another embodiment, the particle size of the millable grinding compound is substantially reduced but not as small as the milled metaxalone.

### Milling bodies

In the method of the present invention, the milling bodies are preferably chemically inert and rigid. The term "chemically-inert", as used herein, means that the milling bodies do not react chemically with the metaxalone or the grinding matrix. The milling bodies are essentially resistant to fracture and erosion in the milling process.

The milling bodies are desirably provided in the form of bodies which may have any of a variety of smooth, regular shapes, flat or curved surfaces, and lacking sharp or raised edges. For example, suitable milling bodies can be in the form of bodies having ellipsoidal, ovoid, spherical or right cylindrical shapes. Preferably, the milling bodies are provided in the form of one or more of beads, balls, spheres, rods, right cylinders, drums or radius-end right cylinders (i.e., right cylinders having hemispherical bases with the same radius as the cylinder). The milling bodies desirably have an effective mean particle diameter (i.e. "particle size") between about 0.1 and 30 mm, more preferably between about 1 and about 15 mm, still more preferably between about 3 and 10 mm.

The milling bodies may comprise various substances such as ceramic, glass, metal or polymeric compositions, in a particulate form. Suitable metal milling bodies are typically spherical and generally have good hardness (i.e. RHC 60-70), roundness, high wear resistance, and narrow size distribution and can include, for example, balls fabricated from type 52100 chrome steel, type 316 or 440C stainless steel or type 1065 high carbon steel.

Preferred ceramics, for example, can be selected from a wide array of ceramics desirably having sufficient hardness and resistance to fracture to enable them to avoid being chipped or crushed during milling and also having sufficiently high density. Suitable densities for milling bodies can range from about 1 to 15 g/cm^{3,}, preferably from about 1 to 8 g/cm³. Preferred ceramics can be selected from steatite, aluminum oxide, zirconium oxide, zirconia-silica, yttria-stabilized zirconium oxide, magnesia-stabilized zirconium oxide, silicon nitride, silicon carbide, cobalt-stabilized tungsten carbide, and the like, as well as mixtures thereof.

Preferred glass milling bodies are spherical (e.g. beads), have a narrow size distribution, are durable, and include, for example, lead-free soda lime glass and borosilicate glass. Polymeric milling bodies are preferably substantially spherical and can be selected from a wide array of polymeric resins having sufficient hardness and friability to enable them to avoid being chipped or crushed during milling, abrasion-resistance to minimize attrition resulting in contamination of the product, and freedom from impurities such as metals, solvents, and residual monomers. Preferred polymeric resins, for example, can be selected from crosslinked polystyrenes, such as polystyrene crosslinked with divinylbenzene, styrene copolymers, polyacrylates such as polymethylmethacrylate, polycarbonates, polyacetals, vinyl chloride polymers and copolymers, polyurethanes, polyamides, high density polyethylenes, polypropylenes, and the like. The use of polymeric milling bodies to grind materials down to a very small particle size (as opposed to mechanochemical synthesis) is disclosed, for example, in U.S. patents 5,478,705 and 5,500,331. Polymeric resins typically can have densities ranging from about 0.8 to 3.0 g/cm³. Higher density polymeric resins are preferred. Alternatively, the milling bodies can be composite particles comprising dense core particles having a polymeric resin adhered thereon. Core particles can be selected from substances known to be useful as milling bodies, for example, glass, alumina, zirconia silica, zirconium oxide, stainless steel, and the like. Preferred core substances have densities greater than about 2.5 g/cm³. In some cases the milling bodies are formed from a ferromagnetic substance, thereby facilitating removal of contaminants arising from wear of the milling bodies by the use of magnetic separation techniques.

Each type of milling body has its own advantages. For example, metals have the highest specific gravities, which increase grinding efficiency due to increased impact energy. Metal costs range from low to high, but metal contamination of final product can be an issue. Glasses are advantageous from the standpoint of low cost and the availability of small bead sizes as low as 0.004 mm. However, the specific gravity of glasses is lower than other media and significantly more milling time is required. Finally, ceramics are advantageous from the standpoint of low wear and contamination, ease of cleaning, and high hardness.

### Agglomerates of biologically active material after processing

Agglomerates comprising particles of biologically active material, said particles having a particle size within the ranges specified above, should be understood to fall within the scope of the present invention, regardless of whether the agglomerates exceed the ranges specified above.

Agglomerates comprising particles of biologically active material, said agglomerates having a total agglomerate size within the ranges specified above, should be understood to fall within the scope of the present invention.

Agglomerates comprising particles of biologically active material, should be understood to fall within the scope of the present invention if at the time of use, or further processing, the particle size of the agglomerate is within the ranges specified above.

Agglomerates comprising particles of biologically active material, said particles having a particle size within the ranges specified above, at the time of use, or further processing, should be understood to fall within the scope of the present invention, regardless of whether the agglomerates exceed the ranges specified above.

### Processing Time

Preferably, the metaxalone and the grinding matrix are dry milled for the shortest time necessary to form the mixture of the metaxalone at the desired particle size in the grinding matrix while minimizing any possible contamination from the media mill and/or the plurality of milling bodies.

Suitable rates of agitation and total milling times are adjusted for the type and size of milling apparatus as well as the milling media, the weight ratio of the other material in the mill (e.g., metaxalone, milling media, etc.) to the plurality of milling bodies, the chemical and physical properties of the grinding matrix, and other parameters that may be optimized empirically.

### Inclusion of the grinding matrix with the biologically active material and separation of the grinding matrix from the biologically active material

In a preferred aspect, the grinding matrix is not separated from the metaxalone but is maintained with the biologically active material in the final product. Preferably the grinding matrix is considered to be Generally Regarded as Safe (GRAS) for pharmaceutical products.

In an alternative aspect, the grinding matrix is separated from the metaxalone. In one aspect, where the grinding matrix is not fully milled, the unmilled grinding matrix is separated from the metaxalone. In a further aspect, at least a portion of the milled grinding matrix is separated from the metaxalone. Any portion of the grinding matrix may be removed, including but not limited to 10%, 25%, 50%, 75%, or substantially all, of the grinding matrix. In some embodiments of the invention, a significant portion of the grinding matrix may comprise particles of a size similar to and/or smaller than the metaxalone particles. Advantageously, the step of removing at least a portion of the grinding matrix from the biologically active material may be performed through means such as selective dissolution, washing, or sublimation.

An advantageous aspect of the invention would be the use of grinding matrix that has two or more components where at least one component is water soluble and at least one component has low solubility in water. In this case washing can be used to remove the matrix component soluble in water leaving the metaxalone in the remaining matrix components.

The metaxalone and the grinding matrix may be combined with one or more pharmaceutically acceptable carriers, as well as any desired excipients or other like agents commonly used in the preparation of medicaments.

The grinding matrix can include in addition to the millable grinding compound and surfactant can include sotehr materials such as: diluents, polymers, binding agents, filling agents, lubricating agents, sweeteners, flavouring agents, preservatives, buffers, wetting agents, disintegrants, effervescent agents and agents that may form part of a medicament, including a solid dosage form, or other excipients required for other specific drug delivery, such as the agents and media listed below under the heading *Medicinal and Pharmaceutical Compositions,* or any combination thereof.

Preferably, the milled material (metaxalone and grinding matrix) with or without additional componnents are used to produced unit dosage forms using methods known in the art such as granulation and compaction. In the unit dosage forms the metaxalone can be present at between about 0.1% and about 99.0% by weight (e.g., about 5% to about 80% by weight, about 10% to about 50% by weight, about 10 to 15% by weight, 15 to 20% by weight, 20 to 25% by weight, 25 to 30% by weight, 30 to 35% by weight, 35 to 40% by weight, 40 to 45% by weight, 45 to 50% by weight, 50 to 55% by weight, 55 to 60% by weight, 60 to 65% by weight, 65 to 70% by weight, 70 to 75% by weight or 75 to 80%)

As used herein "pharmaceutically acceptable carrier" includes any and all solvents, dispersion media, coatings, antibacterial and antifungal agents, isotonic and absorption delaying agents, and the like that are physiologically compatible. Preferably, the carrier is suitable for parenteral administration, intravenous, intraperitoneal, intramuscular, sublingual, pulmonary, transdermal or oral administration. Pharmaceutically acceptable carriers include sterile aqueous solutions or dispersions and sterile powders for the extemporaneous preparation of sterile injectable solutions or dispersion. The use of such media and agents for the manufacture of medicaments is well known in the art. Except insofar as any conventional media or agent is incompatible with the pharmaceutically acceptable material, use thereof in the manufacture of a pharmaceutical composition according to the invention is contemplated.

Pharmaceutical acceptable carriers according to the invention may include one or more of the following examples:
(1) surfactants and polymers,, including, but not limited to polyethylene glycol (PEG), polyvinylpyrrolidone (PVP), polyvinylalcohol, crospovidone, polyvinylpyrrolidone-polyvinylacrylate copolymer, cellulose derivatives, hydroxypropylmethyl cellulose, hydroxypropyl cellulose, carboxymethylethyl cellulose, hydroxypropyllmethyl cellulose phthalate, polyacrylates and polymethacrylates, urea, sugars, polyols, and their polymers, emulsifiers, sugar gum, starch, organic acids and their salts, vinyl pyrrolidone and vinyl acetate; and or
(2) binding agents such as various celluloses and cross-linked polyvinylpyrrolidone, microcrystalline cellulose; and or
(3) filling agents such as lactose monohydrate, lactose anhydrous, microcrystalline cellulose and various starches; and or
(4) lubricating agents such as agents that act on the flowability of the powder to be compressed, including colloidal silicon dioxide, talc, stearic acid, magnesium stearate, calcium stearate, silica gel; and or
(5) sweeteners such as any natural or artificial sweetener including sucrose, xylitol, sodium saccharin, cyclamate, aspartame, and accsulfame K; and or
(6) flavouring agents; and or
(7) preservatives such as potassium sorbate, methylparaben, propylparaben, benzoic acid and its salts, other esters of parahydroxybenzoic acid such as butylparaben, alcohols such as ethyl or benzyl alcohol, phenolic chemicals such as phenol, or quarternary compounds such as benzalkonium chloride; and or
(8) buffers; and or
(9) Diluents such as pharmaceutically acceptable inert fillers, such as microcrystalline cellulose, lactose, dibasic calcium phosphate, saccharides, and/or mixtures of any of the foregoing; and or
(10) wetting agents such as corn starch, potato starch, maize starch, and modified starches, croscarmellose sodium, crosspovidone, sodium starch glycolate, and mixtures thereof; and or
(11) disintegrants; and or
(12) effervescent agents such as effervescent couples such as an organic acid (e.g., citric, tartaric, malic, fumaric, adipic, succinic, and alginic acids and anhydrides and acid salts), or a carbonate (e.g. sodium carbonate, potassium carbonate, magnesium carbonate, sodium glycine carbonate, L-lysine carbonate, and arginine carbonate) or bicarbonate (e.g. sodium bicarbonate or potassium bicarbonate); and or
(13) other pharmaceutically acceptable excipients.

The dosage forms are suitable for use in animals and in particular in man typically and are chemically stable under the conditions of manufacture and storage. The medicaments comprising metaxalone are formulated as a solid. In another embodiment, the metaxalone, optionally together with the grinding matrix or at least a portion of the grinding matrix, may be combined into a medicament with another biologically active material, or even additional metazalone that differs in median particle size. In the latter embodiment, a medicament may be achieved which provides for different release characteristics - early release from the milled metaxalone material, and later release from a larger average size metaxalone.

### Pharmacokinetic Properties of Submicron Metaxalone Compositions

### Smaller Tmax

In some case the metaxalone compositions exhibit a smaller Tₘₐₓ than conventional fromulations (e.g., Skealxin). In one example, the metaxalone composition has a Tₘₐₓ (under fasted conditions) of less than about 3.5 hours, less than about 3 hours, less than about 2.75 hours, less than about 2.5 hours, less than about 2.25 hours, less than about 2 hours, less than about 1.75 hours, less than about 1.5 hours, less than about 1.25 hours, less than about 1.0 hours, less than about 50 minutes, less than about 40 minutes, or less than about 30 minutes.

### Increased Bioavailability

In some cases, the metaxalone compositions exhibit increased dose-normalized bioavailability (AUC) and thus require smaller doses as compared to a conventional composition (e.g.,Skelaxin). Any drug composition can have adverse side effects. Thus, lower doses of drugs which can achieve a similar or better therapeutic effect as those observed with larger doses of conventional compositions are desired.

### Reduced Food Effect

In some cases, the pharmacokinetic profile of the metaxalone compositions is less affected by the fed or fasted state of a subject ingesting the composition than is the pharmacokinetic profile of a conventional formulation (e.g., Skelaxin). This means that there is reduced difference in the quantity of composition or the rate of composition absorption when the compositions are administered in the fed versus the fasted state. Thus, the compositions of the invention reduce or substantially eliminate the effect of food on the pharmacokinetics of the composition.

In some cases, the increase in Cmax of the metaxalone compositions of the invention, when administered in the fed versus the fasted state, is less than about 35% greater, less than about 30% greater, less than about 25% greater, less than about 20% greater, less than about 15% greater or less than about 10% greater. This is an especially important feature in treating patients with difficulty in maintaining a fed state.

In some cases the metaxalone compositions have a Tₘₐₓ under fed conditions that does not substantially differ from the Tₘₐₓ under fasted conditions. Thus, the Tₘₐₓ under fed conditions is less than 130%, less than 120%, less than 110% or less than 105% of the Tₘₐₓ under fasted conditions.

Benefits of a dosage form which reduces the effect of food include an increase in subject convenience, thereby increasing subject compliance, as the subject does not need to ensure that they are taking a dose either with or without food. Other benefits may include less variability of the Cmax or AUC due to the effect of food on the absorption of the drug and where side effects a dose related, less side effects.

A preferred metaxalone composition of the invention exhibits in comparative pharmacokinetic testing with a standard conventional drug active composition, in oral suspension, capsule or tablet form, a Tₘₐₓ which is less than about 100%, less than about 90%, less than about 80%, less than about 70%, less than about 60%, less than about 50%, less than about 40%, or less than about 30%, of the Tₘₐₓ exhibited by the standard conventional drug active composition (e.g., Skelaxin).

In addition, preferably the dose-normalized Cₘₐₓ of a metaxalone composition of the invention is greater than the Cₘₐₓ of a conventional drug active composition. A preferred composition of the invention exhibits in comparative pharmacokinetic testing with a standard conventional drug active composition (e.g., Skelaxin), a dose-normalized Cₘₐₓ which is greater than about 70%, greater than about 80%, greater than about 90%, greater than about 100%, greater than about 110%, greater than about 120%, greater than about 130%, greater than about 140%, greater than about 150% greater than about 160%, greater than about 170% greater than about 180% , greater than about 200%, greater than about 250% greater than about 300% of than the Cₘₐₓ exhibited by the standard conventional drug active composition.

In addition, preferably the metaxalone composition has a dose-normalized AUC greater than that of the equivalent conventional composition. A preferred composition of the invention exhibits in comparative pharmacokinetic testing with a standard conventional drug active composition (e.g. Skelaxin), a dose-normalized AUC which is greater than about 110%, greater than about 120%, greater than about 130%, greater than about 140%, greater than about 150%, greater than about 160%, greater than about 170% , or greater than about 180% of the AUC exhibited by the standard conventional drug active composition.

Any standard pharmacokinetic protocol can be used to determine blood plasma concentration profile in humans following administration of a composition, and thereby establish whether that composition meets the pharmacokinetic criteria set out herein. For example, a randomized single-dose crossover study can be performed using a group of healthy adult human subjects. The number of subjects should be sufficient to provide adequate control of variation in a statistical analysis, and is typically about 10 or greater, although for certain purposes a smaller group can suffice. Each subject receives by oral administration at time zero a single dose (e.g., 300 mg) of a test formulation of composition, normally at around 8 am following an overnight fast. The subjects continue to fast and remain in an upright position for about 4 hours after administration of the composition. Blood samples are collected from each subject prior to administration (e.g., 15 minutes) and at several intervals after administration. For the present purpose it is preferred to take several samples within the first hour, and to sample less frequently thereafter. Illustratively, blood samples could be collected at 15, 30, 45, 60, and 90 minutes after administration, then every hour from 2 to 10 hours after administration. Additional blood samples may also be taken later, for example at 12 and 24 hours after administration. If the same subjects are to be used for study of a second test formulation, a period of at least 7 days should elapse before administration of the second formulation. Plasma is separated from the blood samples by centrifugation and the separated plasma is analyzed for composition by a validated high performance liquid chromatography (HPLC) or liquid chromatography mass spectrometry (LCMS) procedure. Plasma concentrations of composition referenced herein are intended to mean total concentrations including both free and bound composition.

Any formulation giving the desired pharmacokinetic profile is suitable for administration according to the present methods. Exemplary types of formulations giving such profiles are liquid dispersions and solid dose forms of composition. If the liquid dispersion medium is one in which the composition has very low solubility, the particles are present as suspended particles. Thus, a metaxalone composition of the invention, upon administration to a subject, provides improved pharmacokinetic and/or pharmacodynamic properties compared with a standard reference indomethacin composition as measured by at least one of speed of absorption, dosage potency, efficacy, and safety.

### Therapeutic uses

Therapeutic uses of the medicaments include pain relief, particularly pain relief for acute, painful musculoskeletal conditions.

### Example 1: Dry milling of metaxalone

Chemically, metaxalone is 5-[3,5-dimethylphenoxy) methyl]-2-oxazolidone. The empirical formula is C₁₂H₁₅NO₃, which corresponds to a molecular weight of 221.25 g/mol. Metaxalone is a white to almost white, odorless crystalline powder freely soluble in chloroform, soluble in methanol and in 96% ethanol, but practically insoluble in ether or water. The mechanism of action of metaxalone in humans has not been established, but may be due to general central nervous system depression.

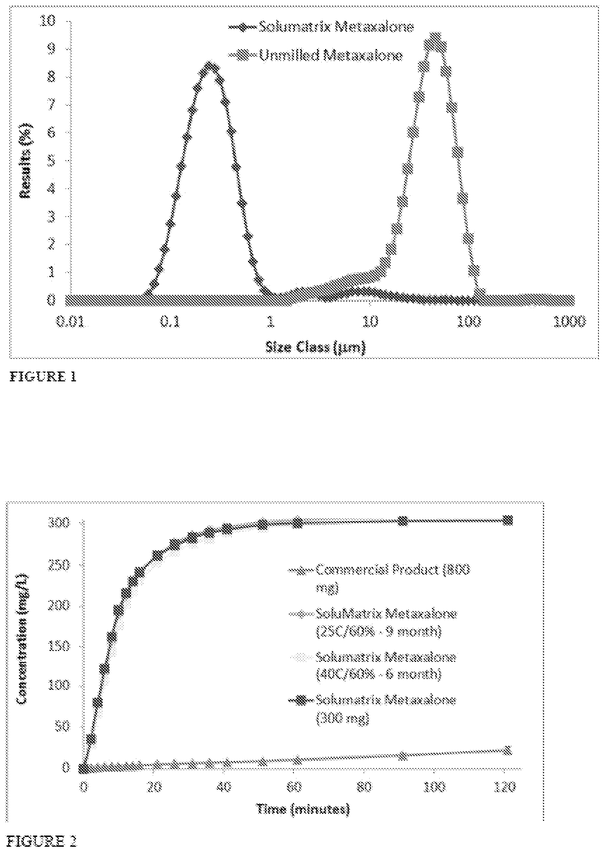

Submicron sized metaxalone drug particles were prepared by dry milling metaxalone drug substance (40%) together with lactose monohydrate and sodium lauryl sulfate in an attritor mill containing stainless steel grinding media. The total batch size was approximately 1 kg. Milled powder was discharged out the bottom of the mill and collected for analysis and further processing. The size distribution of the milled metaxalone particles was measured using a Malvern Mastersizer 3000 laser particle size analyzer equipped with a Hydro MV liquid sample cell module containing an aqueous dispersing medium. Table 1, below, includes size data for the milled and unmilled metaxalone. The milled metaxalone showed a significant reduction in particle size relative to the unmilled metaxalone. The Dv10, Dv50, and Dv90 of the milled metaxalone each show a >100 fold decrease in magnitude relative to the unmilled metaxalone (Figure 1, Table 1).

**Table 1**

| | **Specific Surface Area (m²/kg)** | **D[4,3] (µm)** | **Dv10 (µm)** | **Dv50 (µm)** | **Dv90 (µm)** |
|---|---|---|---|---|---|
| Unmilled Metaxalone | 199.7 | 47.3 | 16.0 | 43.3 | 81.8 |
| Milled Metaxalone | 22500.0 | 0.816 | 0.128 | 0.269 | 0.616 |

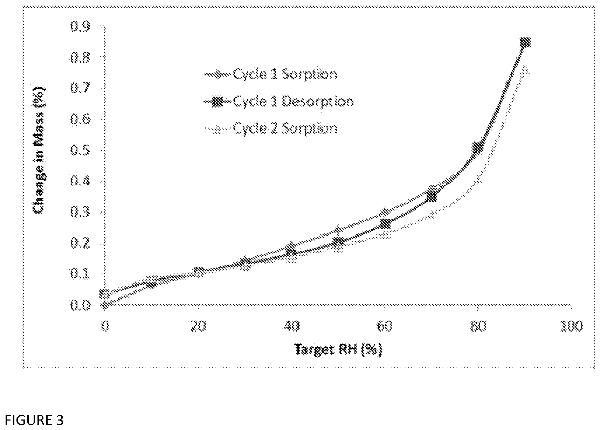

Moisture uptake of milled powder was studied by exposing the sample to a constant temperature of 40°C and varying the relative humidity from cycles of 0% to 90% to 0% using a SMS Dynamic Vapor Sorption Analyzer. Dynamic vapor sorption (DVS) showed less than 0.9% moisture uptake (Figure 3) and little to no hysteresis between sorption and desorption curves indicating only surface absorption with little or no bulk absorption. DVS analysis also gave no indication of amorphous content.

### Example 2: Preparation and characterization of submicron metaxalone tablets

Milled powder was compressed into tablets with a dry granulation process. Briefly, the milled powder was blended with binder, disintegrant, and lubricant, and then converted into free-flowing granules using a roller compaction system (TFC-Lab Micro, Freund Vector). These granules were blended with additional disintegrant, binder, and lubricant and compressed to yield tablets of 300 mg potency. These tablets were tested for dissolution at an initial time point and at 2 weeks and 4 weeks. Stability conditions were 25°C/60%RH and 40°C/75%RH. The results of this analysis are depicted in Figure 2. Dissolution was compared to 800 mg Skelaxin tablets. Dissolution was done in a Sotax Dissolution Apparatus with 1000 ml of 0.01 N HCl (pH=2) at 37°C using Type 2 Apparatus (paddle) set to a rotational speed of 100 rpm. Aliquots of the dissolution test solutions were filtered and analyzed using an in-line UV spectrophotometer at a detection wave length of 271 nm. Dissolution of the metaxalone Submicron tablets (Figure 2) showed that 100% of the dose is dissolved in the first 60 minutes. This is in contrast to the 800 mg Skelaxin product (commercial product) which shows that less than 2% (10.8 ± 0.3 mg) of the drug is dissolved in the first 60 minutes. This result demonstrates the improved performance of Submicron tablets as compared to commercial Skelaxin tablets. Dissolution of the Submicron metaxalone tablets shows no difference after 2 and 4 weeks under 25°C/60%RH and 40°C/75%RH conditions (Figure 2).

Content uniformity was measured on ten Submicron 300 mg tablets and demonstrated a % drug content of 98.5% of label claim and an acceptance value of 2.39 indicating a uniform distribution of drug between tablets. Impurity studies were done on both tablets and milled powder. No significant increase in impurities was seen over the 4 week stability study (Table 2).

| Table 2: Trace Metals Analysis (Tablets) | | | | | |
|---|---|---|---|---|---|
| | Cr (ppm) | Mn (ppm) | Ni (ppm) | Mo (ppm) | Fe (ppm) |
| Report 1 | 5 | 1 | 2 | 1 | 19 |
| Report 2 | 4 | 1 | 2 | 1 | 17 |
| Specification³ | ≤ 25 ppm | ≤ 250 ppm | < 25 ppm | < 25 ppm | < 1300 ppm |

### Example 3: Pharmacokinetic testing of 300 mg and 600 mg sub micron formulation metaxalone

Pharmcokinetic testing of metaxalone Submicron formulation tablets containing 300 mg of metaxalone was carried out. Healthy Subjects were administered one (300mg dose) or two (600mg dose) tablets. A summary of the pharmacokinetic parameters is present in Table 3.

**Table 3: Summary of Pharmcokinetic Parameters**

| **Parameter (Unit)** | **Statistic** | **Submicron formulation 300 mg fasted** | **Submicron formulation 600 mg fasted** | **Skelaxin 800 mg fasted** | **Submicron formulation 600 mg fed** |
|---|---|---|---|---|---|
| | | **(N = 20)** | **(N = 20)** | **(N = 20)** | **(N = 20)** |
| | | | | | |
| AUC₀₋ₜ (h•ng/mL) | N | 20 | 20 | 20 | 20 |
| Arithmetic | Arithmetic Mean | 8309.189 | 20979.872 | 13469.045 | 16954.968 |
| | SD | 3156.683 | 7558.413 | 7910.617 | 5860.618 |
| | Geometric CV % | 46.6 | 35.7 | 73.3 | 35.6 |
| | Geometric Mean | 7646.011 | 19803.463 | 11311.445 | 16028.488 |
| | | | | | |
| AUC_{0-∞} (h•ng/mL) | N | 20 | 20 | 13 | 20 |
| Arithmetic | Arithmetic Mean | 8560.987 | 21499.877 | 16687.346 | 17398.831 |
| | SD | 3189.367 | 7780.329 | 8947.791 | 6047.867 |
| | Geometric CV% | 45.7 | 35.8 | 56.2 | 35.8 |
| | Geometric Mean | 7901.380 | 20287.23 | 14706.165 | 16437.798 |
| | | | | | |
| Cₘₐₓ (ng/mL) | N | 20 | 20 | 20 | 20 |
| Arithmetic | Arithmetic Mean | 2577.393 | 4825.295 | 1744.503 | 4383.555 |
| | SD | 917.210 | 1505.835 | 1006.140 | 1683.359 |
| | Geometric CV% | 43.3 | 29.3 | 59.3 | 39.3 |
| | Geometric Mean | 2395.117 | 4630.751 | 1510.936 | 4092.624 |
| | | | | | |
| Tₘₐₓ (h) | N | 20 | 20 | 20 | 20 |
| | Arithmetic Mean | 1.478 | 2.503 | 4.283 | 2.340 |
| | SD | 0.693 | 0.857 | 1.605 | 1.206 |
| | Median | 1.500 | 2.500 | 4.500 | 2.250 |
| | Minimum | 0.500 | 1.000 | 1.500 | 0.750 |
| | Maximum | 2.500 | 4.000 | 8.050 | 5.000 |
| | | | | | |
| T_{½} (h) | N | 20 | 20 | 13 | 20 |
| | Arithmetic Mean | 1.569 | 1.769 | 7.298 | 1.774 |
| | SD | 0.305 | 0.381 | 2.404 | 0.302 |
| | Geometric CV% | 21.3 | 23.1 | 33.3 | 18.2 |
| | Geometric Mean | 1.538 | 1.727 | 6.951 | 1.748 |

| | | | | | |
|---|---|---|---|---|---|
| Arithmetic Mean calculated as sum of observations/N; Geometric CV% calculated as 100*sqrt[(exp(SD²)-1] where SD is the standard deviation of the log-transformed values; Geometric Mean calculated as Nth root of (product of observations). N = number of subjects included in the pharmacokinetic population for each treatment; AUC₀₋ₜ = area under the plasma concentration-time curve from time 0 to the time of the last quantifiable concentration; AUC_{0-∞} = area under the plasma concentration-time curve from time 0 extrapolated to infinite time; Cₘₐₓ = maximum plasma concentration; Tₘₐₓ = time of maximum plasma concentration; T_{½} = terminal elimination half-life. | | | | | |

Analysis of the relative bioavailability shows there was a statistically significant difference in the
relative bioavailability of the Submicron Metaxalone tablets at doses of 300 and 600 mg compared with Skelaxin® 800 mg tablet for all parameters compared. The Submicron Metaxalone tablets at a dose of 300 mg was more bioavailable than the Skelaxin® 800 mg tablet,
with respect to rate of exposure (Cmax). The Submicron Metaxalone tablets at a dose of 600 mg was more bioavailable than the Skelaxin® 800 mg tablet, with respect to rate and extent of exposure (Cmax and AUC). The Submicron Metaxalone tablets at a dose of 300 mg compared with a dose of 600 mg indicate a statistically significant difference for relative bioavailability with respect to all parameters with exception of T½. The non-parametric analysis for Tmax showed the three treatments to be statistically significantly different.

Bioequivalence analysis of the Submicron Metaxalone tablets at a dose of 300 mg compared with the Skelaxin® 800 mg tablet indicated that the products were not bioequivalent. The geometric mean ratios (GMRs) [90% CI] for AUC0-t and AUC0-∞ were 0.677 [0.587; 0.780] and 0.555 [0.506; 0.610], respectively. The GMR for Cmax was 1.625 [1.403; 1.883] indicating that the peak exposure for the Submicron Metaxalone tablets (1 x 300 mg tablet) was significantly higher than that of the Skelaxin® 800 mg tablet, but extent of exposure was significantly lower for the Submicron Metaxalone tablets. Bioequivalence analysis of the Submicron Metaxalone tablets at a dose of 600 mg compared with the Skelaxin® 800 mg tablet indicated that the products were not bioequivalent. The GMRs [90% CI] for AUC0-t and AUC0-∞ were 1.824 [1.583; 2.102] and 1.484 [1.351; 1.631], respectively. The GMR for Cmax was 3.259 [2.813; 3.776] indicating that the extent and rate of exposure for the Submicron Metaxalone tablets at a dose of 600 mg (2 x 300 mg tablets) was significantly higher than that of the Skelaxin® 800 mg tablet.

There was evidence of a food effect for the Submicron Metaxalone tablets with respect to rate and extent of absorption, expressed as Cmax and AUC. The GMRs [90% CI] for AUC0-t and AUC0-∞ were 0.809 [0.752; 0.871] and 0.810 [0.753; 0.871], respectively. The GMR for Cmax was 0.884 [0.768; 1.017]. Results indicated that food decreased Cmax by approximately 12% (p=0.1446) and approximately 20% for AUC (p<0.0001). The Tmax was comparable for the Submicron Metaxalone tablets administered with food compared to the Submicron Metaxalone tablets administered fasted.

Analysis of the coefficients of variation of the Submicron 300mg dose and Submicron 600mg dose with the Skelaxin 800 mg releaved that the Submircon dosage forms exhibited less pharmacokinetic variability. These results are presented in Tables 4-6.

**Table 4: AUC₀₋ₜ for All Subjects (geometric means and coefficients of variation)**

| Test article | , ng·h/mL (CV) | % reduction in CV relative to Skelaxin |
|---|---|---|
| Submicron 300 mg fasted | 7646.011 (46.6) | 36% |
| Submicron 600 mg fasted | 19803.463 (35.7) | 56% |
| Skelaxin 800 mg fasted | 11311.445 (73.3) | N/A |
| Submicron 600 mg fed | 16028.488 (35.6) | 51% |

**Table 5: AUC_{0-inf} for All Subjects (geometric means and coefficients of variation)**

| Test article | AUC_{0-inf}, ng·h/mL (CV) | % reduction in CV relative to Skelaxin |
|---|---|---|
| Submicron 300 mg fasted | 7901.380 (45.7) | 19% |
| Submicron 600 mg fasted | 20287.23 (35.8) | 36% |
| Skelaxin 800 mg fasted | 14706.165 (56.2) | N/A |
| Submicron 600 mg fed | 16437.798 (35.8) | 36% |

**Table 6: Cₘₐₓ for All Subjects (geometric means and coefficients of variation)**

| Test article | Cₘₐₓ, ng/mL (CV) | % reduction in CV relative to Skelaxin |
|---|---|---|
| Submicron 300 mg fasted | 2395.117 (43.3) | 27% |
| Submicron 600 mg fasted | 4630.751 (29.3) | 51% |
| Skelaxin 800 mg fasted | 1510.936 (59.3) | N/A |
| Submicron 600 mg fed | 4092.624 (39.3) | 34% |

When compared by gender, Submicron 300mg dose and ubmicron 600mg dose showed no clinicall relvent differneces between male and female subjects. This is in contrast to Skelaxin 800mg where, according to the prescribing information (Spetember 2011), "bioavailability of metaxalone was significantly higher in females compared to males as evidenced by Cmax (2115 ng/mL) versus 1335 ng/mL) and AUC0-inf (17884 ng·hr/mL versus 10328 ng·h/mL)". In addition, the mean half-life of Skelanin was reported 11.1 hours in females and 7.6 hours in males and the apparent volume of distribution of metaxalone was approximately 22% higher in males than in females, but not significantly different when adjusted for body weight. Comparative data in shown in Tables 7 and 8.

**Table 7: Cₘₐₓ Gender Comparison**

| Test article | Cₘₐₓ (ng/mL) | | Female/Male Ratio |
|---|---|---|---|
| | females | males | |
| Skelaxin 800 mg² | 2115 | 1335 | 1.58 |
| Submicron metaxalone 300 mg fasted¹ | 2396.132 | 2798.933 | 0.86 |
| Submicron metaxalone 600 mg fasted¹ | 5059.845 | 4538.622 | 1.11 |
| Submicron metaxalone 600 mg fed¹ | 3970.391 | 4888.533 | 0.81 |

| | | | |
|---|---|---|---|
| ¹Data from clinical study ²Data from Skelaxin Prescribing Information dated 9/2011. | | | |

**Table 8: AUC_{0-inf} Gender Comparison**

| Test article | AUC_{0-inf}(ng·h/mL) | | Female/Male Ratio |
|---|---|---|---|
| | females | males | |
| Skelaxin 800 mg² | 17884 | 10328 | 1.73 |
| Submicron metaxalone 300 mg fasted¹ | 8454.366 | 8691.302 | 0.97 |
| Submicron metaxalone 600 mg fasted¹ | 22456.397 | 20330.797 | 1.10 |
| Submicron metaxalone 600 mg fed¹ | 17090.510 | 17775.667 | 0.96 |

| | | | |
|---|---|---|---|
| ¹Data from clinical study ²Data from Skelaxin Prescribing Information dated 9/2011. | | | |

### Overall Summary of Pharmcokinetic Data

Analysis of the relative bioavailability of the Submicron Metaxalone tablets at a dose of 300 mg and Skelaxin® 800 mg tablet indicate that the Submicron Metaxalone tablets were more bioavailable than the Skelaxin® tablet with respect to rate of absorption and the Submicron Metaxalone tablets at a dose of 600 mg were significantly more bioavailable than the Skelaxin® tablet for rate and extent of absorption.

The T½ for the Submicron Metaxalone tablet (doses of 300 mg and 600 mg) was significantly shorter than for the Skelaxin® tablet (800 mg) administered under fasted conditions.

Non-parametric analysis of Tmax showed the treatments to be significantly different.

The Submicron Metaxalone tablets (1 x 300 mg tablet) versus Skelaxin® 800 mg tablet GMR for Cmax was 1.625 [1.403; 1.883] indicating that the peak exposure for Submicron Metaxalone tablets was significantly higher; however, the extent of exposure was significantly lower for the Submicron Metaxalone tablets. The GMRs [CI] for AUC0-t and AUC0-∞ were 0.677 [0.587; 0.780] and 0.555 [0.506; 0.610], respectively.

The Submicron Metaxalone tablets at a dose of 600 mg versus Skelaxin® 800 mg tablet GMRs [CI] for AUC0-t and AUC0-∞ were 1.824 [1.583; 2.102] and 1.484 [1.351; 1.631], respectively. The GMR for Cmax was 3.259 [2.813; 3.776] indicating that the extent and rate of exposure for the Submicron Metaxalone tablets (2 x 300 mg tablets) was significantly higher than that of the Skelaxin® 800 mg tablet.

There was evidence of a food effect for the Submicron Metaxalone tablets, as GMRs [90% CI] for AUC0-t and AUC0-∞ were 0.809 [0.752; 0.871] and 0.810 [0.753; 0.871], respectively, and for Cmax was 0.884 [0.768; 1.017], indicating that food decreased the rate of absorption by approximately 12% and decreased the extent of absorption by 20%.

The Tmax was comparable for the Submicron Metaxalone tablets administered with food compared to the Submicron tablets administered fasted.

Variability, expressed as the geometric coefficient of variation (CV%), for the PK parameters was approximately 30% to 50% lower for the Submicron Metaxalone treatments compared with the Skelaxin® treatment.

Comparison of the PK parameters by gender showed no clinically relevant differences between male and female subjects, across treatments; results summarized by gender were comparable to the results summarized by treatment alone.

## Claims

1. An oral solid unit dosage form of metaxalone containing 300 mg of metaxalone, lactose monohydrate, and sodium lauryl sulfate,
wherein the metaxalone has a median particle size, determined on a particle volume basis, between 50 and 900 nm, and
wherein the dissolution rate of the metaxalone, when tested in a Sotax Dissolution Apparatus using 1000 ml of 0.01 N HCl (pH=2) at 37°C and Type 2 Apparatus (paddle) set to a rotational speed of 100 rpm, is such that at least 80% dissolves in 60 min.

2. The oral solid unit dosage form of claim 1 wherein the metaxalone has a median particle size, determined on a particle volume basis, between 50 and 800 nm.

3. The oral solid unit dosage form of claim 1, wherein the metaxalone has a median particle size, determined on a particle volume basis, between 50 nm and 700 nm.

4. The oral solid unit dosage form of claim 1, wherein the metaxalone has a median particle size, determined on a particle volume basis, between 50 nm and 500 nm.

5. The oral solid unit dosage form of claim 1, wherein the metaxalone has a median particle size, determined on a particle volume basis, between 100 nm and 400 nm.

6. The oral solid unit dosage form of claim 1 wherein at least 90% of the metaxalone dissolves in 60 min.

7. The oral solid unit dosage form of claim 1, wherein at least 99% of the metaxalone dissolves in 60 min.

8. The oral solid unit dosage form of claim 1, wherein at least 50% of the metaxalone dissolves in 30 min.

9. The oral solid unit dosage form of claim 8, wherein at least 50% of the metaxalone dissolves in 20 min.

10. The oral solid unit dosage form of claim 9, wherein at least 50% of the metaxalone dissolves in 15 min.

11. The oral solid unit dosage form of claim 1, wherein at least 25% of the metaxalone dissolves in 20 min.

12. The oral solid unit dosage form of claim 11, wherein at least 25% of the metaxalone dissolves in 15 min.

13. The oral solid unit dosage form of claim 12, wherein at least 25% of the metaxalone dissolves in 10 min.

14. The oral solid unit dosage form of claim 1 wherein the unit dosage form is a tablet.

15. The oral solid unit dosage form of claim 15, wherein the tablet contains a binder, a disintegrant and a lubricant.

## Patentansprüche

1. Orale feste Einheitsdosisform von Metaxalon, die 300 mg Metaxalon, Lactose-monohydrat und Natriumlaurylsulfat enthält,
wobei das Metaxalon eine mediane Teilchengröße, bestimmt auf einer Teilchenvolumenbasis, zwischen 50 und 900 nm hat und
wobei die Auflösungsrate des Metaxalons, getestet in einem Sotax Dissolution Apparatus mit 1000 ml 0,01 N HCl (pH=2) bei 37°C und mit einem auf eine Umdrehungsgeschwindigkeit von 100 U/min eingestellten Typ-2-Apparat (Paddle), dermaßen ist, dass mindestens 80% innerhalb von 60 min in Lösung geht.

2. Orale feste Einheitsdosisform nach Anspruch 1,
wobei das Metaxalon eine mediane Teilchengröße, bestimmt auf einer Teilchenvolumenbasis, zwischen 50 und 800 nm hat.

3. Orale feste Einheitsdosisform nach Anspruch 1,
wobei das Metaxalon eine mediane Teilchengröße, bestimmt auf einer Teilchenvolumenbasis, zwischen 50 und 700 nm hat.

4. Orale feste Einheitsdosisform nach Anspruch 1,
wobei das Metaxalon eine mediane Teilchengröße, bestimmt auf einer Teilchenvolumenbasis, zwischen 50 und 500 nm hat.

5. Orale feste Einheitsdosisform nach Anspruch 1,
wobei das Metaxalon eine mediane Teilchengröße, bestimmt auf einer Teilchenvolumenbasis, zwischen 100 und 400 nm hat.

6. Orale feste Einheitsdosisform nach Anspruch 1,
wobei mindestens 90% des Metaxalons innerhalb von 60 min in Lösung geht.

7. Orale feste Einheitsdosisform nach Anspruch 1,
wobei mindestens 99% des Metaxalons innerhalb von 60 min in Lösung geht.

8. Orale feste Einheitsdosisform nach Anspruch 1,
wobei mindestens 50% des Metaxalons innerhalb von 30 min in Lösung geht.

9. Orale feste Einheitsdosisform nach Anspruch 8,
wobei mindestens 50% des Metaxalons innerhalb von 20 min in Lösung geht.

10. Orale feste Einheitsdosisform nach Anspruch 9,
wobei mindestens 50% des Metaxalons innerhalb von 15 min in Lösung geht.

11. Orale feste Einheitsdosisform nach Anspruch 1,
wobei mindestens 25% des Metaxalons innerhalb von 20 min in Lösung geht.

12. Orale feste Einheitsdosisform nach Anspruch 11,
wobei mindestens 25% des Metaxalons innerhalb von 15 min in Lösung geht.

13. Orale feste Einheitsdosisform nach Anspruch 12,
wobei mindestens 25% des Metaxalons innerhalb von 10 min in Lösung geht.

14. Orale feste Einheitsdosisform nach Anspruch 1,
wobei es sich bei der Einheitsdosisform um eine Tablette handelt.

15. Orale feste Einheitsdosisform nach Anspruch 15,
wobei die Tablette ein Bindemittel, ein Sprengmittel und ein Schmiermittel enthält.

## Revendications

1. Forme pharmaceutique unitaire solide orale de métaxalone contenant 300 mg de métaxalone, du lactose monohydraté, et du laurylsulfate de sodium,
dans laquelle la métaxalone a une taille de particule médiane, déterminée sur une base de volume de particules, comprise entre 50 et 900 nm, et
dans laquelle le taux de dissolution de la métaxalone, lorsqu'il est évalué dans un appareil de dissolution Sotax en utilisant 1000 ml de HCl 0,01 N (pH=2) à 37 °C et un appareil de type 2 (à pales) réglé à une vitesse de rotation de 100 tours/min, est tel qu'au moins 80 % soient dissous en 60 min.

2. Forme pharmaceutique unitaire solide orale selon la revendication 1 dans laquelle la métaxalone a une taille de particule médiane, déterminée sur une base de volume de particules, comprise entre 50 et 800 nm.

3. Forme pharmaceutique unitaire solide orale selon la revendication 1, dans laquelle la métaxalone a une taille de particule médiane, déterminée sur une base de volume de particules, comprise entre 50 nm et 700 nm.

4. Forme pharmaceutique unitaire solide orale selon la revendication 1, dans laquelle la métaxalone a une taille de particule médiane, déterminée sur une base de volume de particules, comprise entre 50 nm et 500 nm.

5. Forme pharmaceutique unitaire solide orale selon la revendication 1, dans laquelle la métaxalone a une taille de particule médiane, déterminée sur une base de volume de particules, comprise entre 100 nm et 400 nm.

6. Forme pharmaceutique unitaire solide orale selon la revendication 1, dans laquelle au moins 90 % de la métaxalone se dissout en 60 min.

7. Forme pharmaceutique unitaire solide orale selon la revendication 1, dans laquelle au moins 99 % de la métaxalone se dissout en 60 min.

8. Forme pharmaceutique unitaire solide orale selon la revendication 1, dans laquelle au moins 50 % de la métaxalone se dissout en 30 min.

9. Forme pharmaceutique unitaire solide orale selon la revendication 8, dans laquelle au moins 50 % de la métaxalone se dissout en 20 min.

10. Forme pharmaceutique unitaire solide orale selon la revendication 9, dans laquelle au moins 50 % de la métaxalone se dissout en 15 min.

11. Forme pharmaceutique unitaire solide orale selon la revendication 1, dans laquelle au moins 25 % de la métaxalone se dissout en 20 min.

12. Forme pharmaceutique unitaire solide orale selon la revendication 11, dans laquelle au moins 25 % de la métaxalone se dissout en 15 min.

13. Forme pharmaceutique unitaire solide orale selon la revendication 12, dans laquelle au moins 25 % de la métaxalone se dissout en 10 min.

14. Forme pharmaceutique unitaire solide orale selon la revendication 1, la forme pharmaceutique unitaire étant un comprimé.

15. Forme pharmaceutique unitaire solide orale selon la revendication 15, le comprimé contenant un liant, un délitant et un lubrifiant.
